# EUROPÄISCHE PATENTSCHRIFT

(11) **EP 3 562 389 B1**
(45) Veröffentlichungstag und Bekanntmachung des Hinweises auf die Patenterteilung: **26.02.2020**
(21) Anmeldenummer: 18746700.6
(22) Anmeldetag: 27.07.2018
(51) Int. Cl.: A61B 5/00, G06K 9/00, A61B 5/11, A61B 5/1171, A63H 3/00

(54) **NUTZUNG EINES ANIMATRONISCHEN KOPFES ZUR TESTAUTOMATISIERUNG VON KAMERABASIERTEN HEAD-TRACKERN**
USE OF AN ANIMATRONIC HEAD FOR THE TEST AUTOMATION OF CAMERA-BASED HEAD TRACKERS
UTILISATION DE TÊTES ANIMATRONIQUES POUR L'AUTOMATISATION DE TESTS DE TRAQUEURS DE TÊTE UTILISANT DES CAMÉRAS

(30) Priorität: 04.08.2017 DE 102017213551
(43) Veröffentlichungstag der Anmeldung: 06.11.2019
(73) Patentinhaber: Audi AG, 85045 Ingolstadt (DE)
(72) Erfinder: STÖRMER, Andre, 85080 Gaimersheim (DE); HUTZLER, Daniel, 85049 Ingolstadt (DE); MICHL, Marco, 85057 Ingolstadt (DE)
(86) Internationale Anmeldenummer: PCT/EP2018/070436
(87) Internationale Veröffentlichungsnummer: WO 2019/025311

(56) Entgegenhaltungen:
- US-A- 5 900 923
- BASTERIS ANGELO ET AL: "Using FATIMA - A robot mannequin head - For validation of head tracking software", 2016 INTERNATIONAL CONFERENCE ON BIOMEDICAL ENGINEERING (BME-HUST), IEEE, 5. Oktober 2016 (2016-10-05), Seiten 125-129, XP033022095, DOI: 10.1109/BME-HUST.2016.7782104 ISBN: 978-1-5090-1097-4 [gefunden am 2016-12-12]
- SOUMITRY J. RAY ET AL: "Automated Head Pose Estimation of Vehicle Operators", PROCEEDINGS OF THE 20TH INTERNATIONAL SYMPOSIUM ON AUTOMATION AND ROBOTICS IN CONSTRUCTION ISARC 2003 -- THE FUTURE SITE, 29. Juni 2011 (2011-06-29), XP055501133, ISSN: 2413-5844, DOI: 10.22260/ISARC2011/0163 ISBN: 978-90-68-14574-8
- TAKUYA HASHIMOTO ET AL: "Development of the Face Robot SAYA for Rich Facial Expressions", SICE-ICCAS 2006 INTERNATIONAL JOINT CONFERENCE, IEEE, PISCATAWAY, NJ, USA, 1. Oktober 2006 (2006-10-01), Seiten 5423-5428, XP031050509, ISBN: 978-89-950038-4-8
- KARSTEN BERNS ET AL: "Control of facial expressions of the humanoid robot head ROMAN", INTELLIGENT ROBOTS AND SYSTEMS, 2006 IEEE/RSJ INTERNATIONAL CONFERENCE ON, IEEE, PI, 1. Oktober 2006 (2006-10-01), Seiten 3119-3124, XP031006584, ISBN: 978-1-4244-0258-8
- JONG WON KWAK ET AL: "A face robot actuated with artificial muscle based on dielectric elastomer", JOURNAL OF MECHANICAL SCIENCE AND TECHNOLOGY, Bd. 19, Nr. 2, 1. Februar 2005 (2005-02-01), Seiten 578-588, XP055501240, DE ISSN: 1738-494X, DOI: 10.1007/BF02916180
- CHAVES-GONZALEZ J M ET AL: "Detecting skin in face recognition systems: A colour spaces study", DIGITAL SIGNAL PROCESSING, ACADEMIC PRESS, ORLANDO,FL, US, Bd. 20, Nr. 3, 1. Mai 2010 (2010-05-01), Seiten 806-823, XP026979345, ISSN: 1051-2004 [gefunden am 2009-10-23]

## Beschreibung

Die vorliegende Erfindung betrifft ein Verfahren zum Testen eines Sensorsystems, welches eingerichtet ist, eine Gesichtsbewegung eines Fahrzeuginsassen in einem Fahrzeug zu erfassen.

In autonom fahrenden Fahrzeugen werden häufig kamerabasierte Head-Tracking-Systeme eingesetzt. In vielen Fällen beinhalten solche Systeme Kameras. Mithilfe von Algorithmen aus den aufgenommenen Bilddaten der Kameras können Parameter über eine Kopfposition, Kopfrotation und oft auch Daten zu Augenöffnung oder Blickrichtung gewonnen werden. In der Regel werden Testpersonen eingesetzt, um Algorithmen für die Head-Tracking-Systeme zu entwickeln oder zu verbessern. Die Ausgabe des Algorithmus hängt maßgeblich von den eingegebenen Bilddaten und somit von der Bewegung und dem Aussehen einer Testperson ab.

Damit ergibt sich der Nachteil, dass das Verhalten wie auch das Aussehen der Testpersonen einen Einfluss auf die Entwicklung der Tracking-Systeme haben. Durch diese Abhängigkeit vom Verhalten beziehungsweise Aussehen der Testpersonen wird ein Vergleich zwischen verschiedenen Erfassungssystemen oder auch zwischen verschiedenen Entwicklungsständen von Software und Hardware nur schwer möglich. Selbst wenn die Testpersonen angehalten werden, ihre Bewegungen beziehungsweise ihr Verhalten möglichst exakt zu reproduzieren, so gelingt dies jedoch nur bedingt. Zudem können Testpersonen nicht für alle Tests herangezogen werden. So ist es beispielsweise unrealistisch, einen Test mit einer Testperson ununterbrochen 10 Stunden lang auszuführen.

Die Offenlegungsschrift DE 10 2013 004 494 A1 beschreibt eine Vorrichtung zur Ermittlung wenigstens eines Funktionsparameters einer Kopfraumheizung eines Kraftfahrzeugsitzes. Diese Vorrichtung umfasst einen wenigstens den Kopf-und Schulterbereich einer Person abbildenden Dummy, ein sitzseitig angeordnetes Befestigungsgestell für den Dummy, sowie eine Rauchgaserzeugungseinrichtung, die mit einem sitzseitig vorgesehenen Einlass lösbar verbindbar ist. Über den Einlass zugeführtes Rauchgas strömt zu wenigstens einer Auslassöffnung, die auf den Kopf- oder Schulterbereich ausgerichtet ist. Ein oder mehrere Sensormittel, welche am Dummy und/oder extern angeordnet sind erfassen wenigstens einen Funktionsparameter während der Umströmung des Dummys mit Rauchgas.

Die Offenlegungsschrift DE 10 2015 210 090 A1 betrifft ein Verfahren zur Kalibrierung eines Systems zur Beobachtung des Kopfes des Fahrers eines Kraftfahrzeugs. Die aktuelle Kalibrierung des Fahrerbeobachtungssystems wird zumindest zu Beginn einer Fahrt des Kraftfahrzeugs überprüft. Daraufhin wird eine Kalibrierung des Systems in einer den Fahrbetrieb des Kraftfahrzeugs nicht gefährdenden Situation durchgeführt. Dies erfolgt dann, wenn der Überprüfungsschritt eine unzureichende Kalibrierung sowie die Notwendigkeit einer erneuten Kalibrierung feststellt.

Die Offenlegungsschrift DE 10 2012 209 664 A1 beschreibt eine Vorrichtung zum Kalibrieren eines ersten Tracking-Systems. Diese Vorrichtung umfasst eine Schnittstelle und eine Kalibrierungseinheit. Die Kalibriereinheit ist dafür eingerichtet, basierend auf jeder für eine Vielzahl der Positionierung eines künstlichen Körpers bestimmten ersten Positionsdaten und basierend auf jeder für eine Vielzahl von Positionierung des künstlichen Körpers bestimmten zweiten Positionsdaten eine Transformationsregel zu bestimmen. Diese Transformationsregel dient zur Transformation von weiteren von dem ersten Tracking-System erhaltenen Positionsdaten, die sich auf das erste Koordinatensystem oder ein weiteres drittes Koordinatensystem beziehen.

BASTERIS ANGELO ET AL: "Using FATIMA - A robot mannequin head - For validation of head tracking software",2016 INTERNATIONAL CONFERENCE ON BIOMEDICAL ENGINEERING (BME-HUST), IEEE, 5. Oktober 2016 (2016-10-05), Seiten 125-129, offenbart einen künstlichen Kopf zur Validierung von Head-Tracking-Software.

SOUMITRY J. RAY ET AL: "Automated Head Pose Estimation of Vehicle Operators",PROCEEDINGS OF THE 20TH INTERNATIONAL SYMPOSIUM ON AUTOMATION AND ROBOTICS IN CONSTRUCTION ISARC 2003 -- THE FUTURE SITE, 29. Juni 2011 (2011-06-29), offenbart die Nutzung eines künstlichen Kopfes zur Kalibrierung eines Modells eines Fahrzeuginsassen.

Aus dem Stand der Technik ergibt sich für die Anmelderin kein Hinweis, dass die Entwicklung und Bereitstellung von Tracking-Systemen auf einem einheitlichen Standard basiert. Es existieren zwar Hilfsmittel, wie zum Beispiel ein Kunstkopf, jedoch sind diese Hilfsmittel nur statisch ausgeführt.

Die vorliegende Erfindung stellt sich die Aufgabe, reproduzierbare Testszenarien bereitzustellen und identische Eingangsparameter zur Kalibrierung von Sensorsystemen, insbesondere für Head-Tracking-Systeme zu erzeugen.

Diese Aufgabe wird durch die vorliegende Erfindung gemäß Patentanspruch 1 gelöst. Vorteilhafte Weiterbildungen dieser Erfindung ergeben sich aus den Unteransprüchen. Ferner stellt die vorliegende Erfindung eine Prüfvorrichtung gemäß Anspruch 7 zur Lösung der Aufgabe bereit.

Die Erfindung sieht ein Verfahren zum Testen eines Sensorsystems, welches eingerichtet ist, eine Gesichtsbewegung eines Fahrzeuginsassen in einem Fahrzeug zu erfassen. Dazu sieht die Erfindung die Ausführung folgender Schritte vor. Zunächst wird ein menschenähnlicher künstlicher Kopf bereitgestellt. Dieser menschenähnliche künstliche Kopf kann aus Kunststoff, Holz, Metall oder anderen Materialien sowie eine Mischung dergleichen geformt sein. Vorzugsweise ist der menschenähnliche, künstliche Kopf maßstabsgetreu ausgebildet. Dies kann hilfreich sein, realistische Kopf- und/oder Gesichtsbewegungen zu erzeugen. Sensorsysteme können damit noch gezielter auf die Erkennung von Menschen getestet und/oder entwickelt werden. Im weiteren Verlauf wird eine mit dem menschenähnlichen künstlichen Kopf verbundene Animationseinrichtung bereitgestellt. Diese Animationseinrichtung kann einen oder mehrere Motoren aufweisen. Diese Motoren können mechanisch oder elektrisch angetrieben werden. So kann beispielsweise die Animationsvorrichtung mehrere Elektromotoren aufweisen. Auch eine Kombination von Elektromotoren und andersgearteten Motoren ist möglich.

Mittels der Animationseinrichtung wird eine vorgegebene Gesichtsbewegung des menschenähnlichen künstlichen Kopfes ausgeführt. Eine Kopfbewegung kann beispielsweise eine Rotation des gesamten Kopfes um seine Symmetrieachse sein. Auch eine Drehung in eine andere Richtung, wie zum Beispiel ein Nicken, ist als Kopfbewegung möglich. Verschiedene Gesichtsbewegungen können beispielsweise durch ein Augenzwinkern, Schließen eines Augenlids, öffnen oder schließen eines Mundes des menschenähnlichen künstlichen Kopfes als Gesichtsbewegungen ermöglicht werden. Diese vorgegebene Gesichtsbewegung werden von dem Sensorsystem erfasst. Somit kann gemäß dieser Erfindung ein sogenannter "animatronischer" Kopf bereitgestellt werden, der einheitliche, standardisierte und exakt identische Gesichtsbewegungen erzeugen kann.

Der Begriff "animatronisch" leitet sich aus dem Englischen "animatronic" ab. Damit werden oft mechanisch, pneumatisch und elektronisch gesteuerte Figuren bezeichnet, die tierähnlich oder anthropomorph sein können. Solche Figuren werden oft in Vergnügungsparks oder auf Jahrmärkten eingesetzt, um für Unterhaltung oder Belustigung zu sorgen. In dieser vorliegenden Anmeldung bezieht sich das Wort "animatronisch" auf den menschenähnlichen, künstlichen Kopf. Dieser animatronische Kopf ist imstande durch die entsprechende Animationseinrichtung Gesichtsbewegungen auszuführen. Diese können gleichförmig durchgeführt werden und so können reproduzierbare Eingangsparameter erzeugt werden. Die Gesichtsbewegungen können wiederholt durchgeführt werden und sind dabei identisch. Damit kann ein Hilfsmittel bereitgestellt werden, welches die Entwicklung von Kopf-Tracking-Systemen vereinheitlichen und sogar verbessern kann.

Die Erfindung sieht vor, dass aus der vorgegebenen Gesichtsbewegung, welche das Sensorsystem erfasst, ein erster Wert abgeleitet wird, wobei dieser erste Wert mit einem entsprechenden zweiten Wert verglichen wird. Dieser erste Wert beschreibt insbesondere ein Resultat, wie das Sensorsystem die vorgegebene Gesichtsbewegung des animatronischen Kopfs erfasst hat.

Eine weitere nicht unter die Anspüche fallende Variante sieht vor, dass der zweite Wert vom menschenähnlichen, künstlichen Kopf bereitgestellt wird. Der erste Wert und der zweite Wert beschreiben in dieser Ausführung die Kopfposition, Kopf-und/oder Gesichtsbewegung. Der erste Wert wird vom Sensorsystem bereitgestellt oder erzeugt. Der zweite Wert kann in einer nicht unter die Ansprüche fallenden Ausführungsform von dem menschenähnlichen künstlichen Kopf bereitgestellt oder generiert werden. Somit kann die vom animatronischen Kopf durchgeführte Gesichtsbewegung durch den zweiten Wert repräsentiert werden. Durch einen Vergleich des ersten Werts mit dem zweiten Wert kann getestet werden, wie gut das Sensorsystem die vorgegebene Bewegung oder eine Position des menschenähnlichen, künstlichen Kopfs erkennt. Dadurch, dass durch den menschenähnlichen künstlichen Kopf bzw. den animatronischen Kopf exakt gleiche reproduzierbare Gesichtsbewegungen möglich werden, können zur Entwicklung oder Kalibration von Sensorsystemen stets dieselben zweiten Werte bereitgestellt werden. Anders formuliert kann der menschenähnliche, künstliche Kopf durch seine Fähigkeit, identische Bewegungen auszuführen, mehrere zweite Werte erzeugen, die jedoch gleich sind. Dadurch kann sichergestellt werden, dass beim Vergleichen mit dem Sensorsystem eine identische Vergleichsgrößen, die identischen zweiten Werte, vorliegen.

Damit wäre es möglich, eine Kamera mit einem unbekannten Algorithmus, eine sogenannte "Blackbox-Kamera", zu testen. Mit Hilfe des menschenähnlichen künstlichen Kopfs können identische reproduzierbare Referenzbewegungen, in diesem Zusammenhang auch als Ground-Truth bekannt, definiert und vorgegeben werden. Diese Referenzbewegungen spiegeln sich in dem zweiten Wert wieder. Eine solche Referenzbewegung kann zum Beispiel eine Drehung des Kopfs um 30 Grad sein. Bei einem Vergleichstest kann geprüft werden, ob das Sensorsystem diese Kopfdrehung zuverlässig detektiert und wenn ja wie gut. Die Qualität der Erkennung ergäbe sich durch den Vergleich des ersten Werts mit dem zweiten Wert. Betrachten verschiedene Sensorsysteme dieses Szenario, so ist sichergestellt, dass die unterschiedlichen Sensorsysteme dieselbe Situation als Grundlage für den Test haben. Bei einem Einsatz von Testpersonen, welche beispielsweise eine Kopfbewegung oder ein Einschlafen simulieren, kann nicht gewährleistet werden, dass die Ausführungen ihrer Gesichtsbewegungen stets dieselben sind. Dieser Nachteil kann durch den animatronischen Kopf beseitigt werden.

Erfindungsgemäß wird der zweite Wert von einem weiteren Sensorsystem, welches die vorgegebene Gesichtsbewegung erfasst, bereitgestellt. Das weitere Sensorsystem kann auch als zweites Sensorsystem bezeichnet werden. Das weitere oder zweite Sensorsystem kann beispielsweise von einem neuen Hersteller bezogen worden sein und sich von dem ursprünglichen Sensorsystem unterscheiden. Beide Sensorsysteme erfassen dazu vorzugsweise den animatronischen Kopf und stellen daraufhin den ersten und zweiten Wert bereit. Wurde zum Beispiel das ursprünglichen Sensorsystem weiterentwickelt und verbessert, so kann das zweite Sensorsystem direkt mit dem ursprünglichen Sensorsystem verglichen werden. Somit können unterschiedliche Sensorsysteme getestet und untereinander verglichen werden.

So wird beispielsweise ein Einschlafen des Fahrers durch den animatronischen Kopf simuliert Dieses Szenario kann von dem künstlichen Kopf beziehungsweise von dem animatronischen Kopf immer wieder und exakt gleich wiedergegeben werden. Die ersten und zweiten Werte würden in diesem Fall den Einschlafvorgang beschreiben, zum Beispiel anhand der Geschwindigkeit, mit der die Augenlider schließen. Weitere Sensorsysteme, welche den menschenähnlichen künstlichen Kopf betrachten, der in diesem Fall einen Einschlafvorgang simuliert, erzeugen gegebenenfalls mehrere zweite Werte. Diese zweiten Werte können mit dem ersten Wert verglichen werden, was einen Vergleich verschiedener Sensorsysteme untereinander ermöglicht. Beispielsweise können verschiedene Kamerasysteme hinsichtlich ihrer Fähigkeit, einschlafende Fahrer rechtzeitig zu erkennen, getestet werden.

Eine weitere Variante sieht vor, dass am menschenähnlichen künstlichen Kopf ein künstliches Auge angeordnet ist und die Animationseinrichtung eine vorgegebene Augenbewegung als die Gesichtsbewegung durchführt. Eine solche Augenbewegung kann beispielsweise durch eine Drehung eines künstlichen Augapfels erfolgen. Auch kann die Augenbewegung durch eine Veränderung der Größe und oder Position der Pupille erreicht werden. Somit können unterschiedliche Reaktionen eines menschlichen Auges auf Umwelteinflüsse, wie zum Beispiel durch Lichteinstrahlung simuliert werden. Ferner kann durch die Augenbewegung eine veränderte Blickrichtung simuliert werden.

Die Erfindung sieht vor, dass am menschenähnlichen künstlichen Kopf ein Augenlid angeordnet ist und die Animationseinrichtung eine vorgegebene Bewegung des Augenlids als die Gesichtsbewegung ausführt. Wird das Auge durch das Augenlid aus einem Oberlid und Unterlid beispielsweise durch ein Zusammenführen des Oberlids mit dem Unterlid geschlossen, so kann damit eine einschlafende Person nachgestellt werden. Werden hingegen das Oberlid und das Unterlid auseinandergezogen, so kann damit eine menschliche Schocksituation nachgestellt werden. Es ist also möglich, bestimmte Ausnahmezustände zu simulieren, welche das Sensorsystem erkennen soll. Durch einheitlich bereitgestellte Bewegungen der Augen beziehungsweise der Augenlider kann ein Sensorsystem einheitlich entwickelt oder kalibriert werden.

Eine weitere Ausführungsform sieht vor, dass am menschenähnlichen künstlichen Kopf ein Gebiss mit einem Kiefer angeordnet ist und die Animationseinrichtung ausgestaltet ist, eine vorgegebene Bewegung des Gebisses durchzuführen. Wird beispielsweise ein Mund schlagartig so weit geöffnet, dass das Gebiss sowie der Kiefer zu sehen ist, so kann dies eine Gesichtsbewegung darstellen, welche auf einen Ausnahmezustand hindeuten kann. Auch diese Bewegung kann der menschenähnliche künstliche Kopf in exakt gleicher Weise wiederholt ausführen. Dies kann dabei helfen, das Sensorsystem derart zu entwickeln, dass es solche Ausnahmezustände sicher erkennt.

Eine bevorzugte Ausführungsform sieht vor, dass die Animationseinrichtung die vorgegebene Gesichtsbewegung wiederholt ausführt. Zur Entwicklung von Sensorsystemen, welche menschliche Gesichtsbewegungen zuverlässig erfassen sollen, sind wiederholt ausgeführte Gesichtsbewegungen in der Regel von Vorteil. Ein eventuell nicht vollständig entwickeltes Sensorsystem benötigt wiederkehrende Gesichtsbewegungen, um diese detektieren zu können. Dabei ist es sehr vorteilhaft, wenn die Gesichtsbewegungen stets dieselben sind. Dies kann dabei helfen, Sensorsysteme schneller zu entwickeln.

Eine vorteilhafte weitere Variante sieht vor, dass die Animationseinrichtung eine vorgegebene Rotation des menschenähnlichen künstlichen Kopfes ausführt. In einem autonom fahrenden Auto ist beispielsweise die Blickrichtung eines Fahrers von entscheidender Bedeutung. Je nach dem wohin der Fahrer blickt, muss das autonom fahrende Auto eventuell unterschiedliche Aufgaben wahrnehmen. Daher kann es wichtig sein, dass das Sensorsystem zuverlässig die Blickrichtung des Fahrers erkennt. Eine veränderte Blickrichtung des Fahrers kann beispielsweise durch eine Rotation des menschenähnlichen künstlichen Kopfes dargestellt werden. Damit kann durch die Rotation des menschenähnlichen künstlichen Kopfes das Sensorsystem dahingehend trainiert werden, unterschiedliche Blickrichtungen des Fahrers zuverlässig zu erkennen. Würde beispielsweise der Fahrer abgelenkt sein und nicht auf die Verkehrssituation achten, so könnte das Sensorsystem dies erkennen und eventuell ein Signal für eine Steuereinheit bereitstellen. Diese könnte beispielsweise die Geschwindigkeit des Fahrzeugs reduzieren oder andere Maßnahmen zur Erhöhung der Verkehrssicherheit automatisch ergreifen.

Die Erfindung sieht vor, dass die Animationseinrichtung in einer definierten Zeit das Augenlid von einem Anfangszustand in einen Endzustand überführt. Häufig werden zwei Augenlider, nämlich das Oberlid und das Unterlid bei der Darstellung eines müden bzw. ein schlafenden Fahrers verwendet. Eine übermüdete Person weist in der Regel eine andere Augenform auf als eine ausgeschlafene Wache Person. Die übermüdete Person besitzt häufig ein Lidschlussverhalten, welches sich von einer wachen Person in der Regel deutlich unterscheidet. Ein gegenüber einem Normalzustand verlangsamtes Lidschlussverhalten könnte auf eine müde Person hindeuten. Da ein Sensorsystem insbesondere eine übermüdete bzw. ein schlafende Person erkennen soll, ist es vorteilhaft, dies entsprechend mithilfe des künstlichen Kopfes simulieren zu können. Somit wird das Sensorsystem trainiert, müde oder einschlafende Personen zu erkennen.

In einer besonderen Ausgestaltungsform ist vorgesehen, dass die Animationseinrichtung mit einem Servomotor realisiert wird. Als Servomotor werden spezielle Elektromotoren bezeichnet, die die Kontrolle der Winkelposition ihrer Motorwelle sowie der Drehgeschwindigkeit und Beschleunigung erlauben. Mit Servomotoren können zum Beispiel detaillierte Gesichtsbewegungen umgesetzt werden. Sie weisen zudem in vielen Fällen eine höhere Zuverlässigkeit auf als andere Motorenarten, wie zum Beispiel drehende Motoren. Servomotoren sind typischerweise gegenüber Schrittmotoren leichter ansteuerbar. Bei dieser Variante können auch mehrere Servomotoren zur Umsetzung verschiedener Gesichtsbewegungen zum Einsatz kommen. So kann beispielsweise ein erster Servomotor eine Kopfrotation umsetzen, ein zweiter Servomotor könnte zum Beispiel verschiedene Augenbewegungen ausführen und ein dritter Servomotor könnte beispielsweise eine Bewegung eines künstlichen Mundes und des Gebisses umsetzen. Ein oder mehrere Servomotoren können derart zusammenwirken, sodass zusätzlich eine komplexe Bewegung der einzelnen Gesichtsbewegungen dargestellt wird. Die in den vorigen Absätzen beschriebenen Gesichtsbewegungen können somit in einer Kombination ausgeführt werden.

In einer besonders vorteilhaften Ausführungsform ist vorgesehen, dass das Sensorsystem die Gesichtsbewegung anhand einer Bewegung einer künstlichen Haut erfasst, welche auf dem menschenähnlichen künstlichen Kopf angebracht ist. Köpfe von menschlichen Fahrern sind im Normalzustand von einer Haut umgeben. D.h. das Sensorsystem soll in der Realität einen echten menschlichen Kopf mit menschlicher Haut erkennen. Um diesen Faktor bei dem künstlichen Kopf zu berücksichtigen, ist es sehr vorteilhaft, wenn der künstliche Kopf, der in dieser Erfindung zugleich auch animatronsich ist, mit einer künstlichen Haut überzogen ist. Diese künstliche Haut ist vorzugsweise der echten menschlichen Haut sehr ähnlich. Idealerweise ist, wie im Hollywood-Film "Terminator" dargestellt, kein Unterschied zwischen echter menschlicher Haut und der künstlichen Haut feststellbar. Dadurch kann eventuell sichergestellt werden, dass das Sensorsystem die künstliche Haut und nicht bestimmte Bauteile des künstlichen beziehungsweise animatronischen Kopfes detektiert. Insgesamt kann es dadurch gelingen, dass Sensorsystem in Form eines Kopf-Tracking-Systems realistischer zu entwickeln und oder zu kalibrieren.

Die vorliegende Erfindung sieht außerdem eine Prüfvorrichtung mit einem Sensorsystem vor, welches eingerichtet ist, eine Gesichtsbewegung eines Fahrzeuginsassen in einem Fahrzeug zu erfassen, und einem menschenähnlichen künstlichen Kopf. Diese Prüfvorrichtung ist durch eine Animationseinrichtung gekennzeichnet, welche mit dem menschenähnlichen künstlichen Kopf verbunden ist und ausgebildet ist, vorgegebene Gesichtsbewegungen wiederholt auszuführen. Die zum Hauptanspruch 1 genannten Merkmale und Vorteile gelten sinngemäß ebenfalls für diesen Vorrichtungsanspruch.

Erfindungsgemäß ist das Sensorsystem ausgebildet, aus der erfassten vorgegebenen Gesichtsbewegung einen ersten Wert abzuleiten, wobei dieser erste Wert mit einem entsprechenden zweiten Wert verglichen wird. Mithilfe einer Auswerteeinrichtung, die einen Algorithmus beinhalten kann, können verschiedene menschliche Zustände, beispielsweise ein Schockzustand durch rasch aufgerissene Augenlider, ein Einschlafen oder andere detektionswürdige Zustände des Menschen erkannt werden. Solche Zustände können beispielsweise allergische Schocks, Stress oder ähnliches sein.

Eine weiterführende nicht unter die Ansprüche fallende Variante sieht vor, dass der zweite Wert vom menschenähnlichen, künstlichen Kopf bereitgestellt ist. Mit Hilfe des animatronischen Kopfs können bestimmte Kopf- und/oder Gesichtsbewegungen reproduzierbar vorgegeben werden. Damit kann der zweite Wert mittels des menschenähnlichen, künstlichen Kopfs definiert werden. Die Prüfvorrichtung mit dem Sensorsystem zeichnet beispielsweise einen realen Menschen auf und geniert aus diesen aufgenommenen Kopf- und/oder Gesichtsbewegungen zum Beispiel den ersten Wert. Dieser erste Wert kann nun mit dem zweiten Wert, der vom animatronischen Kopf bereitgestellt ist, verglichen werden. Damit kann mittels des animatronischen Kopfs das Sensorsystem hinsichtlich seiner Fähigkeit, bestimmte Zustände eines Menschen zu erkennen, eingeschätzt werden. So kann zum Beispiel festgestellt werden, wie gut das Sensorsystem einen müden, evtl. einschlafenden Fahrer erkennt.

Die Erfindung sieht vor, dass der zweite Wert von einem weiteren Sensorsystem bereitgestellt ist, welches die Gesichtsbewegung des menschenähnlichen künstlichen Kopf erfasst. Damit können insbesondere verschiedene Sensorsysteme untereinander verglichen werden. Ist zum Beispiel das Sensorsystem mit Hilfe des menschenähnlichen künstlichen Kopf ausreichend gut entwickelt hinsichtlich seiner Fähigkeit die Gesichtsbewegungen zu erkennen, so kann dieses Sensorsystem direkt mit weiteren Sensorsystemen verglichen werden.

Eine weitere Variante sieht ein Kraftfahrzeug mit einer Prüfvorrichtung vor. Viele der bereits angeführten Merkmale und Vorteile lassen sich auf diesen Vorrichtungsanspruch sinngemäß übertragen.

Zu den Ausführungsbeispielen gehören auch Weiterbildungen des erfindungsgemäßen Verfahrens, die Merkmale aufweisen, wie sie bereits im Zusammenhang mit den Weiterbildungen des erfindungsgemäßen Verfahrens und/oder Prüfvorrichtung beschrieben worden sind. Aus diesem Grund sind die entsprechenden Weiterbildungen des erfindungsgemäßen Verfahrens hier nicht noch einmal beschrieben.

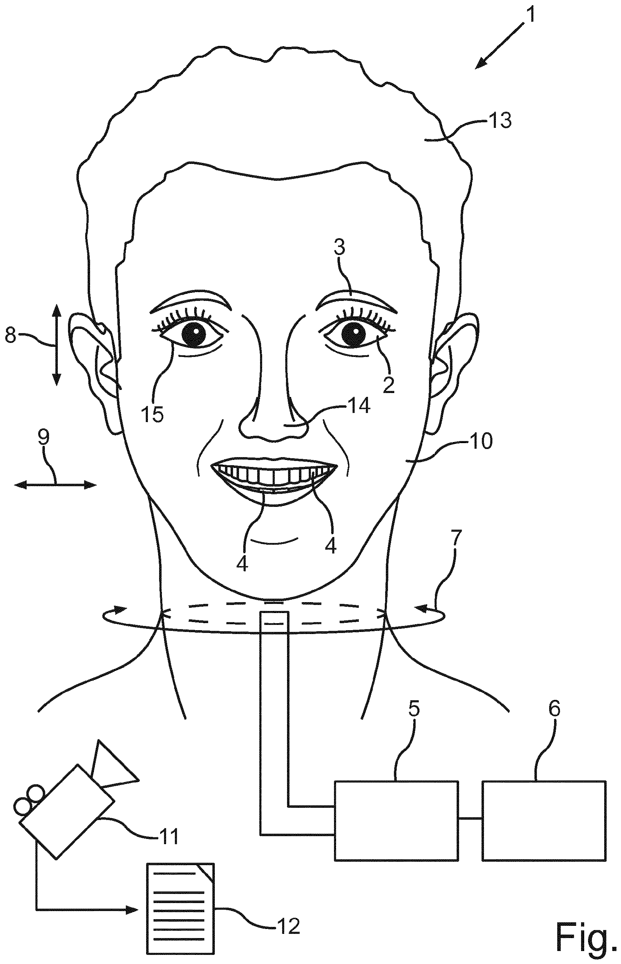

Im Folgenden sind Ausführungsbeispiele der Erfindung beschrieben. Hierzu zeigt die einzige Figur (Fig.) eine schematische Darstellung eines künstlichen Kopfes;

In der Fig. sind funktionsgleiche Elemente jeweils mit denselben Bezugszeichen versehen.

Die Fig. zeigt eine schematische Darstellung eines künstlichen Kopfes 1. Dieser künstliche Kopf 1 ist menschenähnlich ausgeführt. Von besonderem Interesse ist dabei die Vorderseite des künstlichen Kopfes 1, also hauptsächlich das künstliche Gesicht des künstlichen Kopfes 1. Der menschenähnliche künstliche Kopf 1 weist dabei wichtige Merkmale eines realen menschlichen Kopfes auf. Im oberen Bereich des künstlichen Kopfs 1 sind Augen 2, Augenlider 15, Augenbrauen 3 sowie eine Perücke 13 angeordnet. Zwischen den Augen 2 und einem Gebiss 4 ist eine Nase 14 angeordnet. Über einen geöffneten Mund sind beispielhaft Zähne dargestellt, welche Teil des Gebisses 4 sind. Um eine Ähnlichkeit mit einem echten Menschen zu erhöhen, ist der menschenähnliche künstliche Kopf 1 mit einer künstlichen Haut 10 überzogen. Damit kann es gelingen, den künstlichen Kopf 1 möglichst realistisch darzustellen.

Über eine Animationseinrichtung 5 wird der künstliche Kopf 1 definiert bewegt. Dabei können mehrere und oder verschiedene Animationseinrichtungen 5 am künstlichen Kopf 1 angeschlossen sein beziehungsweise innerhalb des künstlichen Kopfes 1 angeordnet sein. In Fig. ist beispielhaft nur eine einzige Animationsvorrichtung 5 dargestellt. Die Animationseinrichtung 5 kann in diesem Beispiel durch eine Steuereinheit 6 vorgegebene Kopf- und/oder Gesichtsbewegungen des künstlichen Kopfs 1 umsetzen. Beispielsweise kann der künstliche Kopf 1 entlang einer Drehrichtung 7 gedreht werden. Somit kann eine vorgegebene Kopfrotation entlang der Drehrichtung 7 realisiert werden. Die Steuereinheit 6 würde in diesem Fall der Animationseinrichtung 5 ein Signal übermitteln. Daraufhin würde die Animationseinrichtung 5 die durch das Signal bestimmte Kopfrotation beispielsweise durch eine aufgesteckt Drehwelle ausführen.

Die Animationseinrichtung 5 kann insbesondere als Servomotor ausgeführt sein. Innerhalb des künstlichen Kopfs 1 angeordnete Animationseinrichtung 5 können zudem weitere Kopf- und/oder Gesichtsbewegungen des künstlichen Kopfs 1 umsetzen. So können beispielsweise die Augenlider 15 entlang einer vertikalen Bewegungsrichtung 8 die Darstellung der Augen 2 gezielt verändern. So kann zum Beispiel vorgegeben werden, dass die Augenlider 15 innerhalb einer vorbestimmten Zeit die Augen 2 schließen. Dies könnte einen einschlafenden Fahrer simulieren. Der simulierte Vorgang des Einschlafens könnte stets exakt gleich ausgeführt werden. Dies könnte durch eine vorgegebene Schließgeschwindigkeit der Augenlider 15 definiert werden. Abweichungen, wie sie zum Beispiel durch den Einsatz von Testpersonen entstehen, können damit deutlich reduziert ausgeschaltet werden.

Ein Schockzustand könnte beispielsweise durch plötzlich weit aufgerissene Augen 2 sowie eine veränderte Form der Augenbrauen 3 nachgestellt werden. Da der künstliche Kopf 1 vorgegebene Kopf- und/oder Gesichtsbewegungen exakt gleich ausführen kann, können damit unterschiedliche Abstufungen von einschlafenden Fahrern bzw. Fahrern im Schockzustand gezielt dargestellt werden. Damit wird die Möglichkeit geschaffen, einen einheitlichen Eingabedatensatz beziehungsweise identische Eingabeparameter für ein Sensorsystem 11 zu erzeugen.

Das Sensorsystem 11 kann insbesondere ein Kamerasystem sein. Dieses Sensorsystem 11 kann die vorgegebenen Kopf- und/oder Gesichtsbewegungen aufzeichnen. Im weiteren Verlauf kann das Sensorsystem 11 eine Ausgabedatei 12 erstellen. Diese Ausgabedatei 12 kann beispielsweise als Videodatei die vom künstlichen Kopf 1 ausgeführten Kopf- und/oder Gesichtsbewegungen beinhalten oder eine Analyse derselben.

Mit Hilfe des animatronischen Kopfs (1) können in Umgebungen mit adaptiver Beleuchtung, sogenannte "closed-loop Systeme" einheitliche Eingabeparameter generiert werden. Eine Videoaufzeichnung des animatronischen Kopf (1) würde aufgrund der möglicherweise unterschiedlichen Beleuchtung zu verschiedenen Video-Dateien führen. Bei einer Bildauswertung wäre zum Beispiel nicht klar, ob ein Unterschied aufgrund einer Gesichtsbewegung oder veränderten Lichtverhältnissen herrührt. Durch den Einsatz des animatronischen Kopf (1) können identische Gesichtsbewegungen ermöglicht werden. Dies kann bei der Entwicklung von kamera-basierten Head-Trackern hilfreich sein.

Häufig ist es erwünscht, einen einschlafenden Fahrer im Fahrzeug im Vorfeld zu erkennen. Da autonom fahrende Fahrzeuge unterschiedliche Autonomiestufen aufweisen können, ist es in der Regel von Bedeutung zu wissen, in welchem Zustand sich der Fahrer befindet. Wenn der Fahrer zum Beispiel schläft, müsste das autonom fahrende Fahrzeug entsprechend mehr Funktionen übernehmen. Bei einem wachen Fahrer, der sich zugleich auf das Verkehrsgeschehen konzentriert, kann eine andere Autonomiestufe sinnvoller sein. Der der künstliche Kopf 1 könnte zum Beispiel durch allmählich zu fallende Augenlider 15 sowie ein Abdriften des künstlichen Kopfs 1 entlang einer horizontalen Bewegungsrichtung 9 erkennen, dass der Fahrer weniger Konzentration aufweist und eventuell im Begriff ist einzuschlafen. Dadurch, dass diese Situation immer wieder und exakt gleich durch den künstlichen Kopf 1 nachgestellt werden kann, kann das Sensorsystem 11 lernen, diese Situation gezielt zu erkennen. Dies kann durch Algorithmen umgesetzt werden, welche einen einschlafenden Fahrer erkennen sollen. Diese Algorithmen können so zuverlässiger entwickelt oder programmiert werden. Die Entwicklung solcher Algorithmen kann durch den künstlichen Kopf 1, der Kopf- und/oder Gesichtsbewegungen immer wieder exakt gleich ausführen kann, erleichtert und verbessert werden.

Zudem ist es möglich, durch den Einsatz des künstlichen Kopfs 1 Testszenarien durchzuführen, welche mit realen Testpersonen nicht durchgeführt werden können. So kann der künstliche Kopf 1 Situationen nachstellen, welche man nicht oder nur schwer mit realen Testpersonen durchführen kann. Dazu zählt zum Beispiel ein Langzeittest über mehrere Stunden, das Nachstellen von Kopf- und/oder Gesichtsbewegungen bei hellem, grellem Lichteinfall, starker Hitze, klirrenden Kälte etc. Es ist schlicht nicht möglich oder erwünscht, echte Testpersonen zum Beispiel in eine Kühlkammer zu setzen, um Kopf- und/oder Gesichtsbewegung bei einem zitternden Menschen zu erhalten. Viele Kopf- und/oder Gesichtsbewegungen sind mit dem künstlichen Kopf 1 darstellbar, welche kaum mit echten Menschen dargestellt werden können.

Auch ist es möglich, gezielte Gesichtsbewegungen, also eine Mimik, durch den künstlichen Kopf 1 darzustellen. Das Sensorsystem 11 kann gezielt auf eine bestimmte Mimik trainiert werden. So ist es beispielsweise möglich, eine vorgegebene Mimik als SOS-Signal für das Sensorsystem 11 zu entwickeln. Dies könnte beispielsweise dann sinnvoll sein, wenn ein Fahrer Opfer einer Entführung wäre und anhand dieser vorgegebenen Mimik das Sensorsystem 11 dies erkennen würde. Damit könnte anhand des erkannten SOS-Signals und ein stiller Notruf abgesetzt werden und/oder Hilfe herbeigerufen werden.

Auch ist es möglich, unterschiedliches menschliches Verhalten durch gezielte Kopf- und/oder Gesichtsbewegungen mit dem künstlichen Kopf 1 abzubilden. So wäre es prinzipiell möglich, einen betrunkenen Fahrer im Vorfeld zu erkennen. Dies zu erkennen, kann für die Steuerung eines autonom fahrenden Fahrzeugs sehr wichtig sein. Ferner ist es möglich, das Sensorsystem 11 derart zu trainieren beziehungsweise zu entwickeln, dass ein Zustand von Kleinkindern oder Babys im Fahrzeug überwacht werden kann.

Durch die künstliche Haut 10 kann der künstliche Kopf 1 realistischer dargestellt werden. So kann das Sensorsystem 11 gezielt entwickelt werden, die künstliche Haut 10 zu erkennen. Darüber hinaus ist es möglich, anhand verschiedener Kopfformen des künstlichen Kopfs 1 und/oder verschiedenen Hauttypen unterschiedliche Köpfe beziehungsweise Gesichter von Menschen abzubilden. So kann zum Beispiel der künstliche Kopf 1 als in einer Ausführung als mitteleuropäischer Kopf und in einer anderen Version als asiatischer Kopf ausgeführt sein. So kann das unterschiedliche Aussehen von Menschen bei der Entwicklung der Sensorsysteme 11 durch entsprechend angepasste künstliche Köpfe 1 berücksichtigt werden.

Insgesamt zeigen die Beispiele, wie eine große Bandbreite von menschlichen Kopf- und/oder Gesichtsbewegungen durch den künstlichen Kopf 1 umgesetzt werden können. Diese Bewegungen können exakt reproduziert werden. Damit stellt der künstliche Kopf 1 ein Werkzeug dar, womit standardisierte und/oder einheitliche Eingangsparameter erzeugt werden können.

Test zum Entwickeln und oder verbessern des Sensorsystems 11 können automatisiert und reproduziert werden. Somit ist es möglich, verschiedene Systeme und Entwicklungsstände objektiv miteinander zu vergleichen. Der menschenähnliche, künstliche Kopf 1 verfügt beispielsweise über eine Recheneinheit, die aus der Kopfposition, Kopf- und/oder Gesichtsbewegungen den zweiten Wert generiert. Dieser zweite Wert kann zum Vergleichen mit dem ersten Wert, den das Sensorsystem 11 bereitstellt, herangezogen werden. Das Sensorsystem 11 kann den ersten Wert durch Erfassen der Bewegungen des animatronischen Kopfs 1 generieren.

Es können verschiedene Sensorsysteme anhand der Bewegungen des animatronischen Kopfs 1 kalibriert werden. Dazu werden vorzugsweise die ersten Werte mit den zweiten Werten verglichen. Die ersten Werte repräsentieren die vom Sensorsystem 11 erfassten Bewegungen oder Position des menschenähnlichen, künstlichen Kopfs 1. Die zweiten Werte stellen in diesem Fall die vom animatronischen Kopf 1 vorgegebenen Positionen oder Bewegungen dar. Zum Beispiel ist durch den animatronischen Kopf 1 ein Lidschlussverhalten der Augen 2 mit einer Schließgeschwindigkeit der Augenlider 15 vorgegeben. Dabei sollen die Augenlider 15 nicht vollständig schließen, sondern im Endzustand ein zur Hälfte geschlossenes Auge darstellen. Dies soll einen einschlafenden Fahrer darstellen. Der animatronische Kopf 1 kann diese Bewegung immer wieder identisch ausführen und mittels seiner Recheneinheit den zweiten Wert bereitstellen. Dieser zweite Wert würde in diesem Fall die langsame Schließbewegung der Augenlider 15 darstellen. Die Bereitstellung des zweiten Werts durch den menschenähnlichen, künstlichen Kopf 1 reduziert im Idealfall die Ausführung der Kopf-und/oder Gesichtsbewegungen auf ein Minimum. Das Sensorsystem 11 zeichnet in diesem Beispiel den animatronischen Kopf 1 auf, erfasst dabei die Kopfposition und dessen Bewegungen und stellt daraus den ersten Wert bereit. Diese Bereitstellung des ersten Werts kann ähnlich erfolgen wie bei dem animatronischen Kopf 1.

Der Unterschied dieser beiden Werte liegt darin, dass der erste Wert die vom Sensorsystem 11 erfassten Positionen und/oder Bewegungen des animatronischen Kopfs 1 darstellt, während der zweite Wert vom animatronischen Kopf 1 direkt bereitgestellt wird. Dadurch ist es möglich, das Sensorsystem 11 durch den Vergleich dieser beiden Werte besser zu beurteilen. Insbesondere kann so ein verbessertes Sensorsystem dadurch erkannt werden, wenn zum Beispiel die Differenz des ersten Werts mit dem zweiten Wert abgenommen hat.

Eine ähnliche Vergleichsmethode ist auch mit einem anderen zweiten Sensorsystem möglich. Im Gegensatz zu dem im vorigen Absatz genannten Beispiel wird in dieser erfindungsgemäßen Variante der zweite Wert ebenfalls von einem Sensorsystem bereitgestellt, wobei in diesem Fall ein weiteres Sensorsystem mit dem vorhandenen Sensorsystem 11 verglichen werden kann. Der erste oder zweite Wert kann zum Beispiel durch eine Rechenoperation, durchgeführt in einer Auswerteeinheit, generiert werden.

Insbesondere können unterschiedliche Kamerasysteme, entwickelt zur Kopf-oder Gesichtserkennung, untereinander getestet und verglichen werden. Verschiedene Sensorsysteme, zum Beispiel von unterschiedlichen Herstellern, können die Kopfposition, Kopf- und/oder Gesichtsbewegung des menschenähnlichen, künstlichen Kopf 1 erfassen und daraus einen ersten bzw. zweiten Wert ableiten. Erfasst man mittels des Sensorsystems 11 und eines zweiten Sensorsystems denselben animatronischen Kopf, so können mittels eines Vergleichs des daraus resultierenden ersten und zweiten Werts diese beiden Sensorsysteme direkt miteinander verglichen werden. In analoger Weise kann der Vergleich mehr als zwei Sensorsysteme umfassen.

Außerdem sind Tests in Umgebungen, die man keiner Testperson zumutet, wie zum Beispiel EMV-Tests, sehr helles Licht, Langzeittests, etc. möglich. Es sind auch Tests möglich, bei denen der Kopf immer weiter rotiert wird, bis das Tracking nicht mehr möglich ist. Zudem können die Kopf- und/oder Gesichtsbewegungen nicht nur qualitativ, sondern quantitativ vorgegeben werden. So kann zum Beispiel der Schließvorgang der Augen 2 durch eine vorgegebene Schließgeschwindigkeit der Augenlider 15 genau im Vorfeld definiert werden. Dies kann dazu beitragen verschiedene Sensorsysteme 11 zu entwickeln oder zu verbessern, welche den Zustand eines Fahrers, insbesondere durch Erkennen von Kopf- und/oder Gesichtsbewegungen, zuverlässiger erkennen können. Durch vorgebbare Kopf- und/oder Gesichtsbewegungen des künstlichen Kopfes 1 können Fehler bei der Entwicklung von Kopf-Tracking-Systemen, die durch die Unterschiede bei realen Testpersonen begründet sind, reduziert werden.

## Patentansprüche

1. Verfahren zum Testen eines Sensorsystems (11), welches eingerichtet ist, eine Gesichtsbewegung eines Fahrzeuginsassen in einem Fahrzeug zu erfassen, durch Ausführen folgender Schritte:
a) Bereitstellen eines menschenähnlichen künstlichen Kopfes (1),
b) Bereitstellen einer mit dem menschenähnlichen künstlichen Kopf (1) verbundenen Animationseinrichtung (5),
c) Ausführen einer vorgegebenen Gesichtsbewegung des menschenähnlichen künstlichen Kopfes (1) mittels der Animationseinrichtung (5) und,
d) Erfassen der vorgegebenen Gesichtsbewegung mit dem Sensorsystem (11), wobei
- aus der mit dem Sensorsystem (11) erfassten vorgegebenen Gesichtsbewegung ein erster Wert abgeleitet wird und dieser erste Wert mit einem entsprechenden zweiten Wert verglichen wird,
**dadurch gekennzeichnet, dass**
- der zweite Wert von einem weiteren Sensorsystem, welches die vorgegebene Gesichtsbewegung erfasst, bereitgestellt wird,
- am menschenähnlichen künstlichen Kopf (1) ein Augenlid (15) angeordnet ist und die Animationseinrichtung (5) eine vorgegebene Bewegung des Augenlids (15) als die Gesichtsbewegung ausführt und
- die Animationseinrichtung (5) in einer definierten Zeit das Augenlid (15) von einem Anfangszustand in einen Endzustand überführt, um ein Einschlafen des Fahrzeuginsassen durch den menschenähnlichen künstlichen Kopf (1) zu simulieren.

2. Verfahren nach Anspruch 1, wobei am menschenähnlichen künstlichen Kopf (1) ein Gebiss (4) mit einem Kiefer angeordnet ist und die Animationseinrichtung (5) ausgestaltet ist, eine vorgegebene Bewegung des Gebisses (4) durchzuführen.

3. Verfahren nach einem der vorhergehenden Ansprüche, wobei die Animationseinrichtung (5) die vorgegebene Gesichtsbewegung wiederholt ausführt.

4. Verfahren nach einem der vorhergehenden Ansprüche, wobei die Animationseinrichtung (5) eine vorgegebene Rotation des menschenähnlichen künstlichen Kopfes (1) ausführt.

5. Verfahren nach einem der vorhergehenden Ansprüche, wobei die Animationseinrichtung (5) mit einem Servomotor realisiert wird.

6. Verfahren nach einem der vorhergehenden Ansprüche, wobei das Sensorsystem (11) die Gesichtsbewegung anhand einer Bewegung einer künstlichen Haut (10) erfasst, welche auf dem menschenähnlichen künstlichen Kopf (1) angebracht ist.

7. Prüfvorrichtung mit
- einem Sensorsystem (11), welches eingerichtet ist, eine Gesichtsbewegung eines Fahrzeuginsassen in einem Fahrzeug zu erfassen,
- einem menschenähnlichen künstlichen Kopf (1),
- einer Animationseinrichtung (5),
- welche mit dem menschenähnlichen künstlichen Kopf (1) verbunden ist und welche
- ausgebildet ist, vorgegebene Gesichtsbewegungen wiederholt auszuführen, wobei
- das Sensorsystem (11) ausgebildet ist, aus mit dem Sensorsystem (11) erfassten vorgegebenen Gesichtsbewegung einen ersten Wert abzuleiten und dieser erste Wert mit einem entsprechenden zweiten Wert verglichen wird,
**dadurch gekennzeichnet, dass**
- der zweite Wert von einem weiteren Sensorsystem bereitgestellt ist, welches die Gesichtsbewegung des menschenähnlichen künstlichen Kopfs (1) erfasst,
- am menschenähnlichen künstlichen Kopf (1) ein Augenlid (15) angeordnet ist und die Animationseinrichtung (5) eine vorgegebene Bewegung des Augenlids (15) als die Gesichtsbewegung ausführt und
- die Animationseinrichtung (5) ausgebildet ist, in einer definierten Zeit das Augenlid (15) von einem Anfangszustand in einen Endzustand überzuführen, um ein Einschlafen des Fahrzeuginsassen durch den menschenähnlichen künstlichen Kopf (1) zu simulieren.

8. Kraftfahrzeug mit einer Prüfvorrichtung nach Anspruch 7.

## Claims

1. Method for testing a sensor system (11), which is set up to detect a facial movement of a vehicle passenger in a vehicle, by implementing the following steps:
a) providing a humanoid imitation head (1),
b) providing an animation device (5) connected to the humanoid imitation head (1),
c) implementing a predetermined facial movement of the humanoid imitation head (1) by means of the animation device (5) and,
d) detecting the predetermined facial movement using the sensor system (11),
wherein
- a first value is derived from the predetermined facial movement detected using the sensor system (11) and this first value is compared to a corresponding second value, **characterised in that**
- the second value is provided by a further sensor system which detects the predetermined facial movement,
- an eyelid (15) is arranged on the humanoid imitation head (1) and the animation device (5) implements a predetermined movement of the eyelid (15) as the facial movement and
- the animation device (5) transfers the eyelid (15) from a start state to an end state in a defined time in order to simulate a falling asleep of the vehicle passenger by the humanoid imitation head (1).

2. Method according to claim 1, wherein a set of teeth (4) with a jaw is arranged on the humanoid imitation head (1) and the animation device (5) is designed to implement a predetermined movement of the set of teeth (4).

3. Method according to any one of the preceding claims, wherein the animation device (5) implements the predetermined facial movement repeatedly.

4. Method according to any one of the preceding claims, wherein the animation device (5) implements a predetermined rotation of the humanoid imitation head (1).

5. Method according to any one of the preceding claims, wherein the animation device (5) is implemented with a servomotor.

6. Method according to any one of the preceding claims, wherein the sensor system (11) detects the facial movement by means of a movement of imitation skin (10) which is attached to the humanoid imitation head (1).

7. Test device having
- a sensor system (11) which is set up to detect a facial movement of a vehicle passenger in a vehicle,
- a humanoid imitation head (1),
- an animation device (5)
- which is connected to the humanoid imitation head (1) and which
- is formed to implement predetermined facial movements repeatedly, wherein
- the sensor system (11) is formed to derive a first value from predetermined facial movement detected using the sensor system (11) and this first value is compared to a corresponding second value,
**characterised in that**
- the second value is provided by a further sensor system which detects the facial movement of the humanoid imitation head (1),
- an eyelid (15) is arranged on the humanoid imitation head (1) and the animation device (5) implements a predetermined movement of the eyelid (15) as the facial movement and
- the animation device (5) is formed to transfer the eyelid (15) from a start state to an end state in a defined time in order to simulate a falling asleep of the vehicle passenger by the humanoid imitation head (1).

8. Motor vehicle having a test device according to claim 7.

## Revendications

1. Procédé pour tester un système de détection (11), lequel est conçu pour détecter un mouvement du visage d'un occupant de véhicule dans un véhicule, par réalisation des étapes suivantes :
a) la fourniture d'une tête humanoïde artificielle (1),
b) la fourniture d'un dispositif d'animation (5) relié à la tête humanoïde artificielle (1),
c) la réalisation d'un mouvement du visage prédéfini de la tête humanoïde artificielle (1) au moyen du dispositif d'animation (5) et,
d) la détection du mouvement du visage prédéfini avec le système de détection (11), dans lequel
- une première valeur est déduite du mouvement du visage prédéfini détecté avec le système de détection (11) et cette première valeur est comparée à une seconde valeur correspondante,
**caractérisé en ce que**
- la seconde valeur est fournie par un autre système de détection, lequel détecte le mouvement du visage prédéfini,
- une paupière (15) est disposée sur la tête humanoïde artificielle (1) et le dispositif d'animation (5) réalise un mouvement prédéfini de la paupière (15) en tant que mouvement du visage et
- le dispositif d'animation (5) amène pendant un temps défini la paupière (15) d'un état initial dans un état final, afin de simuler un endormissement de l'occupant de véhicule à l'aide de la tête humanoïde artificielle (1).

2. Procédé selon la revendication 1, dans lequel une denture (4) avec une mâchoire est disposée sur la tête humanoïde artificielle (1) et le dispositif d'animation (5) est conçu pour réaliser un mouvement prédéfini de la denture (4).

3. Procédé selon l'une quelconque des revendications précédentes, dans lequel le dispositif d'animation (5) reproduit le mouvement du visage prédéfini.

4. Procédé selon l'une quelconque des revendications précédentes, dans lequel le dispositif d'animation (5) réalise une rotation prédéfinie de la tête humanoïde artificielle (1).

5. Procédé selon l'une quelconque des revendications précédentes, dans lequel le dispositif d'animation (5) est réalisé avec un servomoteur.

6. Procédé selon l'une quelconque des revendications précédentes, dans lequel le système de détection (11) détecte le mouvement du visage en s'appuyant sur un mouvement d'une peau artificielle (10), laquelle est appliquée sur la tête humanoïde artificielle (1).

7. Dispositif de contrôle avec
- un système de détection (11), lequel est conçu pour détecter un mouvement du visage d'un occupant de véhicule dans un véhicule,
- une tête humanoïde artificielle (1),
- un dispositif d'animation (5),
- lequel est relié à la tête humanoïde artificielle (1) et lequel
- est conçu pour réaliser à plusieurs reprises des mouvements du visage prédéfinis, dans lequel
- le système de détection (11) est conçu pour déduire du mouvement du visage prédéfini détecté avec le système de détection (11) une première valeur et cette première valeur est comparée à une seconde valeur correspondante,
**caractérisé en ce que**
- la seconde valeur est fournie par un autre système de détection, lequel détecte le mouvement du visage de la tête humanoïde artificielle (1),
- une paupière (15) est disposée sur la tête humanoïde artificielle (1) et le dispositif d'animation (5) réalise un mouvement prédéfini de la paupière (15) en tant que mouvement du visage et
- le dispositif d'animation (5) est conçu pour amener, dans un temps défini, la paupière (15) d'un état initial dans un état final, afin de simuler un endormissement de l'occupant de véhicule à l'aide de la tête humanoïde artificielle (1).

8. Véhicule automobile avec un dispositif d'essai selon la revendication 7.
